# EUROPEAN PATENT SPECIFICATION

(11) **EP 3 199 161 B1**
(45) Date of publication and mention of the grant of the patent: **15.12.2021**
(21) Application number: 15844737.5
(22) Date of filing: 25.09.2015
(51) Int. Cl.: A61K 31/551, A61K 9/08, A61P 27/02, A61P 27/06

(54) **PHARMACEUTICAL PREPARATION**
PHARMAZEUTISCHE ZUBEREITUNG
PRÉPARATION PHARMACEUTIQUE

(30) Priority: 25.09.2014 JP 2014195279
(43) Date of publication of application: 02.08.2017
(73) Proprietor: KOWA COMPANY, LTD., Nagoya-shi, Aichi-ken 460-8625 (JP)
(72) Inventor: ODA, Hiroshi, Fuji-shi Shizuoka 4178650 (JP); SUZUKI, Yuuki, Fuji-shi Shizuoka 4178650 (JP)
(74) Representative: Blodig, Wolfgang
(86) International application number: PCT/JP2015/077015
(87) International publication number: WO 2016/047721

(56) References cited:
- EP-A1- 2 319 514
- EP-A1- 3 199 162
- WO-A1-2010/010715
- JP-A- H0 924 085
- US-A1- 2008 064 681
- SUMI KENGO ET AL: "IOP-lowering effect of isoquinoline-5-sulfonamide compounds in ocular normotensive monkeys", BIOORGANIC & MEDICINAL CHEMISTRY LETTERS, PERGAMON, AMSTERDAM, NL, vol. 24, no. 3, 25 December 2013 (2013-12-25), pages 831-834, XP028819897, ISSN: 0960-894X, DOI: 10.1016/J.BMCL.2013.12.085
- D A Tsarev ET AL: "EVALUATING THE STABILITY OF A CATIONIC PLASTOQUINONE DERIVATIVE (PDTP) IN VISOMITIN EYE DROPS Translated from Khimiko-Farmatsevticheskii", , 4 April 2013 (2013-04-04), pages 40-44, XP055220497, Retrieved from the Internet: URL:http://rd.springer.com/content/pdf/10. 1007/s11094-013-0932-3.pdf [retrieved on 2015-10-13]
- ISOBE, TOMOYUKI ET AL.: 'Effects of K-115, a Rho-Kinase Inhibitor, on Aqueous Humor Dynamics in Rabbits' CURRENT EYE RESEARCH vol. 39, no. 8, pages 813 - 822, XP009501175
- 'Ganka New Insight' TENGAN'YAKU -JOSHIKI TO HIJOSHIKI vol. 2, 10 July 1997, pages 15 - 23, XP009501405
- 'Iyakuhin Interview Form Glanatec(R) Tengan'eki' X. KANRITEKI JIKO NI KANSURU KOMOKU June 2015, page 55, XP009501344

## Description

### [Field of the Invention]

The present invention relates to a pharmaceutical preparation and the like.

### [Background of the Invention]

It is known that halogenated isoquinoline derivatives such as ripasudil (chemical name: 4-fluoro-5-[[(2S)-2-methyl-1,4-diazepan-1-yl]sulfonyl]isoquinoline) represented by the following structural formula: 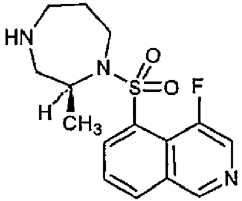 and 4-bromo-5-[[(2S)-2-methyl-1,4-diazepan-1-yl]sulfonyl]isoquinoline represented by the following structural formula: 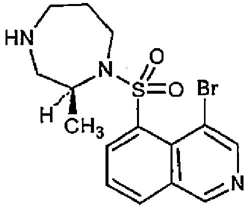 have pharmacological action such as Rho kinase inhibitory action (Patent Literatures 1 and 2, for example), and thus, are usable for the prevention or treatment of eye diseases. Specifically, these halogenated isoquinoline derivatives have been reported to be useful, for example, for the prevention or treatment of ocular hypertension, glaucoma, and the like (Patent Literature 3, for example), or for the prevention or treatment of ocular fundus diseases such as age-related macular degeneration and the like (Patent Literature 4, for example).

Hence, it is extremely useful to establish a technique for producing stable preparations of these halogenated isoquinoline derivatives as ophthalmic agents, for example.

### [Citation List]

### [Patent Literature]

[Patent Literature 1] JP-B-4212149
[Patent Literature 2] WO2006/115244
[Patent Literature 3] WO2006/068208
[Patent Literature 4] JP-B-5557408 The light instability of aqueous solutions of sulfonyl isoquinoline derivatives used for the treatment of glaucoma and ocular hypertension is known, see e.g.EP2319514.

### [Summary of the Invention]

### [Technical Problem]

To produce a preparation of a halogenated isoquinoline derivative, ripasudil, as an ophthalmic agent or the like, the present inventors initially evaluated the photostability of ripasudil. As a result, ripasudil per *se* was revealed to be extremely stable with respect to light. The photostability of an organic compound is believed to be dependent on its structure, rather than its state (such as a solid or a liquid). An ophthalmic agent or the like is generally a composition containing water (aqueous composition), and ripasudil was expected to have no problem with photostability even if it was incorporated into the aqueous composition.

Very unexpectedly, however, as a result of further research conducted by the present inventors, it was revealed that even though ripasudil per *se* is stable with respect to light, when it is incorporated into an aqueous composition, it becomes unstable with respect to light, and exposure to light causes the amount of degradation products to gradually increase.

Accordingly, it is an object of the present invention to provide a technique for improving the stability of a halogenated isoquinoline derivative such as ripasudil with respect to light in an aqueous composition.

### [Solution to Problem]

The present inventors further conducted extensive research to solve the above-described problem, and found that the photodegradation of ripasudil in an aqueous composition is caused by radiation of rays having wavelengths near 300 nm, and when the aqueous composition is stored in a package that blocks rays with these wavelengths, a pharmaceutical preparation having improved photostability can be provided, thus completing the present invention.

In summary, the present invention provides following <1> to <4>.
<1> A pharmaceutical preparation obtained by storing an aqueous composition comprising a compound represented by Formula (1): 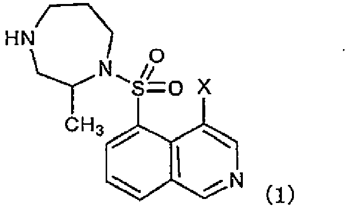
   wherein X represents a halogen atom,
   or a salt thereof, or a solvate of the compound or the salt thereof, in a package that blocks a ray with a wavelength of 300 to 335 nm.
<2> A method for improving photostability of a compound represented by Formula (1), a salt thereof, or a solvate of the compound or the salt thereof in an aqueous composition, the method comprising the step of storing the aqueous composition comprising the compound represented by Formula (1), or a salt thereof, or a solvate of the compound or the salt thereof in a package that blocks a ray with a wavelength of 300 to 335 nm.
<3> An aqueous composition comprising a compound represented by Formula (1), or a salt thereof, or a solvate of the compound or the salt thereof, the aqueous composition being stored in a package that blocks a ray with a wavelength of 300 to 335 nm.
<4> A method for preserving a compound represented by Formula (1), or a salt thereof, or a solvate of the compound or the salt thereof, the method comprising the step of storing an aqueous composition comprising the compound represented by Formula (1), or a salt thereof, or a solvate of the compound or the salt thereof in a package that blocks a ray with a wavelength of 300 to 335 nm.

### [Effects of the Invention]

In accordance with the present invention, the stability of a halogenated isoquinoline derivative such as ripasudil with respect to light in an aqueous composition can be improved.

### [Description of Embodiments]

The present specification discloses, although is in no way limited to, the following embodiments of invention, by way of example.
[1] A pharmaceutical preparation obtained by storing an aqueous composition comprising a compound represented by Formula (1): 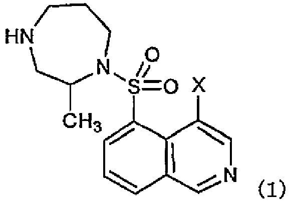
   wherein X represents a halogen atom,
   or a salt thereof, or a solvate of the compound or the salt thereof, in a package that blocks a ray with a wavelength of 300 to 335 nm.
[2] The pharmaceutical preparation according to [1], wherein the compound represented by Formula (1) is ripasudil.
[3] The pharmaceutical preparation according to [1] or [2], wherein the package blocks a ray with a wavelength of 300 to 370 nm.
[4] The pharmaceutical preparation according to [1] or [2], wherein the package blocks a ray with a wavelength of 300 to 395 nm.
[5] The pharmaceutical preparation according to [1] or [2], wherein the package blocks a ray with a wavelength of 270 to 335 nm.
[6] The pharmaceutical preparation according to [1] or [2], wherein the package blocks a ray with a wavelength of 270 to 370 nm.
[7] The pharmaceutical preparation according to [1] or [2], wherein the package blocks a ray with a wavelength of 270 to 395 nm.
[8] The pharmaceutical preparation according to any of [1] to [7], wherein the inside of the package is visible.
[9] The pharmaceutical preparation according to any of [1] to [8], wherein a primary package is:
   a package which is a container containing a substance that prevents transmission of ultraviolet light, the package being preferably a package of which inside is visible, the container being preferably made of plastic, more preferably made of polyolefin-based resin or polyester-based resin and the container being preferably a container for eye drops, and the substance being preferably one or more selected from the group consisting of ultraviolet scattering agents and ultraviolet absorbers, more preferably one or more selected from the group consisting of zinc oxide, titanium oxide and benzotriazole-based ultraviolet absorbers; or
   a package which is a container including a member containing a substance that prevents transmission of ultraviolet light, the package being preferably a package of which inside is visible, the container being preferably made of plastic, more preferably made of polyolefin-based resin or polyester-based resin and the container being preferably a container for eye drops, the member being preferably a heat-shrinkable film (shrinkable film), and the substance being preferably one or more selected from the group consisting of ultraviolet scattering agents and ultraviolet absorbers, more preferably one or more selected from the group consisting of zinc oxide, titanium oxide and benzotriazole-based ultraviolet absorbers.
[10] The pharmaceutical preparation according to any of [1] to [9] including, as a secondary package, one or more selected from the group consisting of:
   a package which is a bag containing a substance that prevents transmission of ultraviolet light, the package being preferably a package of which inside is visible, the bag being preferably made of plastic, more preferably made of polyolefin-based resin or polyester-based resin and the bag being preferably a bag for eye drop instillation, and the substance being preferably one or more selected from the group consisting of ultraviolet scattering agents and ultraviolet absorbers, more preferably one or more selected from the group consisting of zinc oxide, titanium oxide and benzotriazole-based ultraviolet absorbers; and
   a box made of paper.
[11] The pharmaceutical preparation according to any of [1] to [10], wherein the primary package is a container made of polyolefin-based resin.
[12] The pharmaceutical preparation according to any of [1] to [10], wherein the primary package is a container made of polyester-based resin.
[13] A method for improving photostability of a compound represented by Formula (1), a salt thereof, or a solvate of the compound or the salt thereof in an aqueous composition, the method comprising the step of storing the aqueous composition comprising the compound represented by Formula (1), or a salt thereof, or a solvate of the compound or the salt thereof in a package that blocks a ray with a wavelength of 300 to 335 nm.
[14] The method according to [13], wherein the compound represented by Formula (1) is ripasudil.
[15] The method according to [13] or [14], wherein the package blocks a ray with a wavelength of 300 to 370 nm.
[16] The method according to [13] or [14], wherein the package blocks a ray with a wavelength of 300 to 395 nm.
[17] The method according to [13] or [14], wherein the package blocks a ray with a wavelength of 270 to 335 nm.
[18] The method according to [13] or [14], wherein the package blocks a ray with a wavelength of 270 to 370 nm.
[19] The method according to [13] or [14], wherein the package blocks a ray with a wavelength of 270 to 395 nm.
[20] The method according to any of [13] to [19], wherein the inside of the package is visible.
[21] The method according to any of [13] to [20], wherein a primary package is:
   a package which is a container containing a substance that prevents transmission of ultraviolet light, the package being preferably a package of which inside is visible, the container being preferably made of plastic, more preferably made of polyolefin-based resin or polyester-based resin and the container being preferably a container for eye drops, and the substance being preferably one or more selected from the group consisting of ultraviolet scattering agents and ultraviolet absorbers, more preferably one or more selected from the group consisting of zinc oxide, titanium oxide and benzotriazole-based ultraviolet absorbers; or
   a package which is a container including a member containing a substance that prevents transmission of ultraviolet light, the package being preferably a package of which inside is visible, the container being preferably made of plastic, more preferably made of polyolefin-based resin or polyester-based resin and the container being preferably a container for eye drops, the member being preferably a heat-shrinkable film (shrinkable film), and the substance being preferably one or more selected from the group consisting of ultraviolet scattering agents and ultraviolet absorbers, more preferably one or more selected from the group consisting of zinc oxide, titanium oxide and benzotriazole-based ultraviolet absorbers.
[22] The method according to any of [13] to [21], which includes, as a secondary package, one or more selected from the group consisting of:
   a package which is a bag containing a substance that prevents transmission of ultraviolet light, the package being preferably a package of which inside is visible, the bag being preferably made of plastic, more preferably made of polyolefin-based resin or polyester-based resin and the bag being preferably a bag for eye drop instillation, and the substance being preferably one or more selected from the group consisting of ultraviolet scattering agents and ultraviolet absorbers, more preferably one or more selected from the group consisting of zinc oxide, titanium oxide and benzotriazole-based ultraviolet absorbers; and
   a box made of paper.
[23] The method according to any of [13] to [22], wherein the primary package is a container made of polyolefin-based resin.
[24] The method according to any of [13] to [22], wherein the primary package is a container made of polyester-based resin.
[25] The pharmaceutical preparation according to any of [1] to [12], wherein the aqueous composition further contains one or more selected from the group consisting of α1 receptor blockers, α2 receptor agonists, β blockers, carbonic anhydrase inhibitors, prostaglandin F2α derivatives, sympathomimetics, parasympathomimetics, calcium antagonists, and cholinesterase inhibitors.
[26] The pharmaceutical preparation according to any of [1] to [12], wherein the aqueous composition further contains one or more selected from the group consisting of latanoprost, nipradilol, dorzolamide, brinzolamide, and timolol, as well as salts thereof.
[27] The method according to any of [13] to [24], wherein the aqueous composition further contains one or more selected from the group consisting of α1 receptor blockers, α2 receptor agonists, β blockers, carbonic anhydrase inhibitors, prostaglandin F2α derivatives, sympathomimetics, parasympathomimetics, calcium antagonists, and cholinesterase inhibitors.
[28] The method according to any of [13] to [24], wherein the aqueous composition further contains one or more selected from the group consisting of latanoprost, nipradilol, dorzolamide, brinzolamide, and timolol, as well as salts thereof.

Examples of the halogen atom in Formula (1) include a fluorine atom, a chlorine atom, and a bromine atom. In Formula (1), a fluorine atom or a bromine atom is preferred as the halogen atom, and a fluorine atom is particularly preferred.

Further, in Formula (1), the carbon atom forming the homopiperazine ring substituted with the methyl group is an asymmetric carbon atom. As a result, stereoisomerism occurs. The compound represented by Formula (1) includes all the stereoisomers, and may be a single stereoisomer or a mixture of various stereoisomers at any given ratio. Preferred as the compound represented by Formula (1) is a compound having the S configuration as the absolute configuration.

The salt of the compound represented by Formula (1) is not particularly limited as long as it is a pharmacologically acceptable salt, and specific examples of the salt include inorganic acid salts such as hydrochloride, sulfate, nitrate, hydrofluoride, and hydrobromate; and organic acid salts such as acetate, tartrate, lactate, citrate, fumarate, maleate, succinate, methanesulfonate, ethanesulfonate, benzenesulfonate, toluenesulfonate, naphthalenesulfonate, and camphorsulfonate, with hydrochloride being preferred.

The compound represented by Formula (1) or a salt thereof may also be in the form of a hydrate or a solvate such as an alcohol solvate, and is preferably in the form of a hydrate.

Specific examples of the compound represented by Formula (1) or a salt thereof, or a solvate of the compound or the salt thereof include:
ripasudil (chemical name: 4-fluoro-5-[[(2S)-2-methyl-1,4-diazepan-1-yl]sulfonyl]isoquinoline) or a salt thereof, or a solvate of the ripasudil or the salt thereof; and
4-bromo-5-[[(2S)-2-methyl-1,4-diazepan-1-yl]sulfonyl]isoquinoline or a salt thereof, or a solvate of 4-bromo-5-[[(2S)-2-methyl-1,4-diazepan-1-yl]sulfonyl]isoquinoline or the salt thereof.

The compound represented by Formula (1) or a salt thereof, or a solvate of the compound or the salt thereof is preferably ripasudil or a salt thereof, or a solvate or ripasudil or the salt thereof, or 4-bromo-5-[[(2S)-2-methyl-1,4-diazepan-1-yl]sulfonyl]isoquinoline or a salt thereof, or a solvate of 4-bromo-5-[[(2S)-2-methyl-1,4-diazepan-1-yl]sulfonyl]isoquinoline or the salt thereof, more preferably ripasudil or a salt thereof, or a solvate of ripasudil or the salt thereof, still more preferably ripasudil or a hydrochloride thereof, or a hydrate of ripasudil or the hydrochloride thereof, and particularly preferably a ripasudil hydrochloride hydrate (ripasudil monohydrochloride dihydrate) represented by the following structural formula:

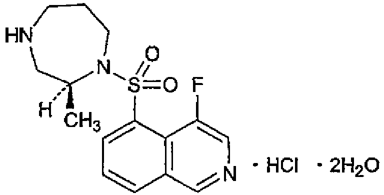

The compound represented by Formula (1) or a salt thereof, or a solvate of the compound or the salt thereof is known and can be produced using a known method. Specifically, ripasudil or a salt thereof, or a solvate of ripasudil or the salt thereof can be produced using the method described in WO1999/020620 or WO2006/057397, for example. 4-Bromo-5-[[(2S)-2-methyl-1,4-diazepan-1-yl]sulfonyl]isoquinoline or a salt thereof, or a solvate of 4-bromo-5-[[(2S)-2-methyl-1,4-diazepan-1-yl]sulfonyl]isoquinoline or the salt thereof can be produced using the method described in WO2006/115244, for example.

The content of the compound represented by Formula (1) or a salt thereof, or a solvate of the compound or the salt thereof in the aqueous composition is not particularly limited, and may be determined as appropriate, in consideration of the target disease, or the sex, age, or symptoms of the patient, for example. From the viewpoint of achieving an excellent pharmacological action, however, the content of the compound represented by Formula (1) or a salt thereof, or a solvate of the compound or the salt thereof is preferably 0.01 to 10 w/v%, more preferably 0.02 to 8 w/v%, and particularly preferably 0.04 to 6 w/v%, calculated as the free form of the compound represented by Formula (1), based on the total volume of the aqueous composition. In particular, when ripasudil is used as the compound represented by Formula (1), from the viewpoint of achieving an excellent pharmacological action, the content of ripasudil or a salt thereof, or a solvate of ripasudil or the salt thereof is preferably 0.05 to 5 w/v%, more preferably 0.1 to 3 w/v%, and particularly preferably 0.1 to 2 w/v%, calculated as the free form, based on the total volume of the aqueous composition.

As used herein, the "aqueous composition" means a composition containing at least water, which may be in the form of a liquid (solution or suspension) or a semisolid (ointment), for example. As the water in the composition, purified water, water for injection, or sterile purified water, for example, can be used.

While the content of water in the aqueous composition is not particularly limited, it is preferably 5 mass% or more, more preferably 20 mass% or more, still more preferably 50 mass% or more, even more preferably 90 mass% or more, and particularly preferably 90 to 99.8 mass%.

The aqueous composition can be prepared into various dosage forms in accordance with known methods described in the General Rules for Preparations in the Japanese Pharmacopoeia 16th Edition, for example. While the dosage form is not particularly limited as long as it can be stored in the below-described package, examples of dosage forms include injections, inhalation solutions, eye drops, eye ointments, ear drops, nasal drops, enemas, liquids for external use, sprays, ointments, gels, oral liquids, and syrups. From the viewpoint of advantageously utilizing the pharmacological action of the compound represented by Formula (1), the dosage form is an agent for an eye disease, which specifically is preferably an eye drop or an eye ointment, and is particularly preferably an eye drop.

The aqueous composition may contain, in addition to the components described above, additives used in drugs, quasi drugs, and the like, in accordance with the dosage form. Examples of such additives include inorganic salts, isotonic agents, chelating agents, stabilizers, pH regulators, antiseptics, antioxidants, thickeners, surfactants, solubilizers, suspending agents, cooling agents, dispersants, preservatives, oily bases, emulsion bases, and water-soluble bases.

Specific examples of these additives include ascorbic acid, potassium aspartate, sodium bisulfite, alginic acid, sodium benzoate, benzyl benzoate, epsilonaminocaproic acid, fennel oil, ethanol, ethylene-vinyl acetate copolymer, sodium edetate, tetrasodium edetate, potassium chloride, calcium chloride hydrate, sodium chloride, magnesium chloride, hydrochloric acid, alkyldiaminoethylglycine hydrochloride solution, carboxyvinyl polymer, dried sodium sulfite, dried sodium carbonate, d-camphor, dl-camphor, xylitol, citric acid hydrate, sodium citrate hydrate, glycerin, gluconic acid, L-glutamic acid, monosodium L-glutamate, creatinine, chlorhexidine gluconate solution, chlorobutanol, sodium dihydrogenphosphate dihydrate, geraniol, sodium chondroitin sulfate, acetic acid, potassium acetate, sodium acetate hydrate, titanium oxide, gellan gum, dibutylhydroxytoluene, potassium bromide, benzododecinium bromide, tartaric acid, sodium hydroxide, polyoxyl 45 stearate, purified lanolin, D-sorbitol, sorbitol solution, sorbic acid, potassium sorbate, taurine, sodium bicarbonate, sodium carbonate hydrate, sodium thiosulfate hydrate, thimerosal, tyloxapol, sodium dehydroacetate, trometamol, concentrated glycerin, mixed tocopherol conceentrate, white petrolatum, mentha water, mentha oil, benzalkonium chloride concentrated solution 50, ethyl parahydroxybenzoate, butyl parahydroxybenzoate, propyl parahydroxybenzoate, methyl parahydroxybenzoate, sodium hyaluronate, human serum albumin, hydroxyethyl cellulose, hydroxypropyl cellulose, hypromellose, glacial acetic acid, sodium pyrosulfite, phenylethyl alcohol, glucose, propylene glycol, bergamot oil, benzalkonium chloride, benzalkonium chloride solution, benzyl alcohol, benzethonium chloride, benzethonium chloride solution, borax, boric acid, povidone, polyoxyethylene (200) polyoxypropylene glycol (70), sodium polystyrene sulfonate, polysorbate 80, polyoxyethylene hydrogenated castor oil 60, partially hydrolyzed polyvinyl alcohol, d-borneol, macrogol 4000, macrogol 6000, D-mannitol, anhydrous citric acid, anhydrous sodium monohydrogen phosphate, anhydrous sodium dihydrogen phosphate, methanesulfonic acid, methylcellulose, 1-menthol, monoethanolamine, aluminum monostearate, polyethylene glycol monostearate, eucalyptus oil, potassium iodide, sulfuric acid, oxyquinoline sulfate, liquid paraffin, borneo camphor, phosphoric acid, dibasic sodium phosphate hydrate, potassium dihydrogenphosphate, sodium dihydrogenphosphate, sodium dihydrogenphosphate monohydrate, malic acid, and petrolatum.

Examples of preferred additives include potassium chloride, calcium chloride hydrate, sodium chloride, magnesium chloride, glycerin, acetic acid, potassium acetate, sodium acetate hydrate, tartaric acid, sodium hydroxide, sodium bicarbonate, sodium carbonate hydrate, concentrated glycerin, hydroxyethyl cellulose, hydroxypropyl cellulose, hypromellose, borax, boric acid, povidone, polysorbate 80, polyoxyethylene hydrogenated castor oil, polyethylene glycol monostearate, partially hydrolyzed polyvinyl alcohol, macrogol 4000, macrogol 6000, anhydrous citric acid, anhydrous sodium monohydrogen phosphate, anhydrous sodium dihydrogen phosphate, methylcellulose, monoethanolamine, phosphoric acid, dibasic sodium phosphate hydrate, potassium dihydrogenphosphate, sodium dihydrogenphosphate, sodium dihydrogenphosphate monohydrate, sodium hyaluronate, glucose, and 1-menthol.

The aqueous composition may further contain, in addition to the components described above, other medicinal components in accordance with the target disease and the like. Examples of such medicinal components include α1 receptor blockers including bunazosin or a salt thereof, or a solvate of bunazosin or the salt thereof, such as bunazosin hydrochloride; α2 receptor agonists including brimonidine or a salt thereof, or a solvate of brimonidine or the salt thereof, such as brimonidine tartrate, and apraclonidine or a salt thereof, or a solvate of apraclonidine or the salt thereof; β blockers including carteolol or a salt thereof, or a solvate of carteolol or the salt thereof, such as carteolol hydrochloride, nipradilol or a salt thereof, or a solvate of nipradilol or the salt thereof, timolol or a salt thereof, or a solvate of timolol or the salt thereof, such as timolol maleate, betaxolol or a salt thereof, or a solvate of betaxolol or the salt thereof, such as betaxolol hydrochloride, levobunolol or a salt thereof, or a solvate of levobunolol or the salt thereof, such as levobunolol hydrochloride, befunolol or a salt thereof, or a solvate of befunolol or the salt thereof, and metipranolol or a salt thereof, or a solvate of metipranolol or the salt thereof; carbonic anhydrase inhibitors including dorzolamide or a salt thereof, or a solvate of dorzolamide or the salt thereof, such as dorzolamide hydrochloride, brinzolamide or a salt thereof, or a solvate of brinzolamide or the salt thereof, acetazolamide or a salt thereof, or a solvate of acetazolamide or the salt thereof, dichlorphenamide or a salt thereof, or a solvate of dichlorphenamide or the salt thereof, and methazolamide or a salt thereof, or a solvate of methazolamide or the salt thereof; prostaglandin F2α derivatives including isopropyl unoprostone or a salt thereof, or a solvate of isopropyl unoprostone or the salt thereof, tafluprost or a salt thereof, or a solvate of tafluprost or the salt thereof, travoprost or a salt thereof, or a solvate of travoprost or the salt thereof, bimatoprost or a salt thereof, or a solvate of bimatoprost or the salt thereof, latanoprost or a salt thereof, or a solvate of latanoprost or the salt thereof, cloprostenol or a salt thereof, or a solvate of cloprostenol or the salt thereof, and fluprostenol or a salt thereof, or a solvate of fluprostenol or the salt thereof; sympathomimetics including dipivefrine or a salt thereof, or a solvate of dipivefrine or the salt thereof, such as dipivefrine hydrochloride, and epinephrine or a salt thereof, or a solvate of epinephrine or the salt thereof, such as epinephrine, epinephrine borate, or epinephrine hydrochloride; parasympathomimetics including distigmine bromide or a salt thereof, or a solvate of distigmine bromide or the salt thereof, pilocarpine or a salt thereof, or a solvate of pilocarpine or the salt thereof, such as pilocarpine, pilocarpine hydrochloride, or pilocarpine nitrate, and carbachol or a salt thereof, or a solvate of carbachol or the salt thereof; calcium antagonists including lomerizine or a salt thereof, or a solvate of lomerizine or the salt thereof, such as lomerizine hydrochloride; and cholinesterase inhibitors including demecarium or a salt thereof, or a solvate of demecarium or the salt thereof, echothiophate or a salt thereof, or a solvate of echothiophate or the salt thereof, and physostigmine or a salt thereof, or a solvate of physostigmine or the salt thereof. One or more of these medicinal components can be incorporated.

Preferred as the other medicinal components is one or more selected from the group consisting of latanoprost, nipradilol, dorzolamide, brinzolamide, and timolol, as well as salts thereof.

The pH of the aqueous composition is not particularly limited, but is preferably 4 to 9, more preferably 4.5 to 8, and particularly preferably 5 to 7. The osmotic pressure ratio of the aqueous composition relative to physiological saline is not particularly limited, but is preferably 0.6 to 3, and particularly preferably 0.6 to 2.

As used herein, the "package" means a package for directly or indirectly storing the aqueous composition. Note that, in the package, a container for directly storing the aqueous composition (for example, a container for eye drops which is to be filled directly with the aqueous composition) is particularly referred to as a "primary package". Additionally, in the package, a package for indirectly storing the aqueous composition (that is, a package for further storing the primary package: for example, a bag for eye drop instillation (a bag for storing the container for eye drops)) is particularly referred to as a "secondary package". The package may be one that can block rays in the range of wavelengths described below in a preserved state generally expected for the pharmaceutical preparation, and may or may not have sealing properties.

Note that the package is a concept that includes all of the "well-closed container", "hermetic container", and "tight container" defined in the General Notices in the Japanese Pharmacopoeia 16th Edition.

In an embodiment where the pharmaceutical preparation includes the secondary package, at least any one of the primary package and secondary package may block rays in the range of wavelengths described below. From the viewpoint of suppressing decomposition of the compound represented by Formula (1) or a salt thereof, or a solvate of the compound or the salt thereof not only during distribution or storage, but also during use, it is preferred that at least the primary package block rays in the range of wavelengths described below.

The form of the package is not particularly limited as long as it can store the aqueous composition, and may be selected or set as appropriate, depending on the dosage form, the use of the pharmaceutical preparation, or whether the package is the primary package or the secondary package, for example. Specific examples of such forms of the primary package include containers for injections, containers for inhalations, containers for sprays, bottle-shaped containers, tubular containers, containers for eye drops, containers for nasal drops, containers for ear drops, and bag containers. Examples of the secondary package include packaging bags (such as bags for eye drop instillation), boxes (such as paper boxes), bottles (such as glass bottles), and cans (such as aluminum cans).

In an embodiment where both the primary package and the secondary package are provided as the package, the primary package is preferably a container for eye drops, and the secondary package is preferably a packaging bag for eye drops, from the viewpoint of advantageously utilizing the pharmacological action of the compound represented by Formula (1).

The material of the package is not particularly limited, and may be selected as appropriate depending on the form of the package. Specific examples of materials include glass, plastics, cellulose, pulp, rubber, and metals.

The material of the primary package for directly storing the aqueous composition is preferably a plastic, for example, from the viewpoint of processability, squeezability, durability, and the like.

The material of the secondary package is preferably a plastic, cellulose, pulp, or paper, for example, from the viewpoint of processability.

When the package is made of a plastic, the resin is preferably a thermoplastic resin, regardless of whether it is a synthetic or natural resin. Specific examples of such resins include polyolefin-based resins, polyester-based resins, polyphenylene ether-based resins, polycarbonate-based resins, polysulfone-based resins, polyamide-based resins, polyvinyl chloride-based resins, and styrene-based resins. One of these resins or a combination of two or more of these resins is preferably used, or a mixture of these resins (polymer alloy) may also be used.

In one embodiment, the material of the primary package is preferably a polyolefin-based resin. As specifically disclosed in Test Examples 7 and 8 below, it was found that although the aqueous composition stored in the primary package may be discolored if it is preserved over a long period at high temperature, when a polyolefin-based resin is used as the material of the primary package, ΔYI is relatively reduced, and excellent stability can be achieved.

Note that in this embodiment, at least a portion of the primary package that contacts the aqueous composition may be formed of a polyolefin-based resin, and a case where a different material is, for example, laminated on the outer side or the like of the primary package also corresponds to the case where "the primary package is made of polyolefin-based resin". As used herein, the expression "made of polyolefin-based resin" means that the polyolefin-based resin is included in at least a portion of the material, and "made of polyolefin-based resin" also includes, for example, a mixture of two or more resins (polymer alloy), which are a polyolefin-based resin and other resins.

The polyolefin-based resin is not particularly limited herein, and may be a polymer of a single monomer (homopolymer) or a copolymer of a plurality of monomers (copolymer). In the case of a copolymer, the mode of polymerization is not particularly limited, and may be random polymerization or block polymerization. Further, the stereoregularity (tacticity) of the polyolefin-based resin is not particularly limited.

Specific examples of such polyolefin-based resins include polyethylene (more specifically, such as low-density polyethylene (including linear low density polyethylene), high-density polyethylene, and mediumdensity polyethylene), polypropylene, cyclic polyolefins, poly(4-methylpentene), polytetrafluoroethylene, ethylene-propylene copolymer, ethylene-α-olefin copolymer, ethylene-acrylic acid copolymer, ethylene-methacrylic acid copolymer, ethylene-vinylacetate copolymer, and ethylene-ethyl acrylate copolymer. These polyolefin-based resins can be used singly or in combination of two or more. As the polyolefin-based resin, polyethylene or polypropylene is preferred, and polypropylene is particularly preferred, from the viewpoint of reducing the ΔYI value.

In another embodiment, the material of the primary package is preferably a polyester-based resin. As specifically disclosed in Test Examples 9 and 10 below, it was found that although preservation of the aqueous composition stored in the primary package at low temperature may result in precipitation of crystals, when a polyester-based resin is used as the material of the primary package, such crystal precipitation is relatively unlikely to occur, and excellent preservation stability can be achieved.

Note that in this embodiment, at least a portion of the primary package that contacts the aqueous composition may be formed of a polyester-based resin, and a case where a different material is, for example, laminated on the outer side or the like of the primary package also corresponds to the case where "the primary package is made of polyester-based resin". As used herein, the expression "made of polyester-based resin" means that the polyester-based resin is included in at least a portion of the material, and "made of polyester-based resin" also includes, for example, a mixture of two or more resins (polymer alloy), which are a polyester-based resin and other resins.

A dicarboxylic acid and a diol forming the polyester-based resin are not particularly limited herein, and examples of dicarboxylic acids include phthalic acid, terephthalic acid, and 2,6-naphthalenedicarboxylic acid, and examples of diols include ethylene glycol, 1,3-propanediol, 1,4-butanediol, 1,4-cyclohexanedimethanol, and bisphenol. The polyester-based resin may be a polymer of a single polyester unit or a polymer of a plurality of polyester units. In the case of a polymer of a plurality of polyester units, the mode of polymerization is not particularly limited, and may be random polymerization or block polymerization. Further, the stereoregularity (tacticity) of the polyester-based resin is not particularly limited.

Specific examples of such polyester-based resins include homopolyesters, for example, polyalkylene terephthalates (such as polyethylene terephthalate and polybutylene terephthalate), polyalkylene naphthalates (such as polyethylene naphthalate and polybutylene naphthalate), polycycloalkylene terephthalates (such as poly(1,4-cyclohexylenedimethylene terephthalate)), and polyarylates (such as a resin composed of bisphenol and phthalic acid); copolyesters containing these homopolyester units as a main component; and copolymers of the above-described homopolyesters. These polyester-based resins can be used singly or in combination of two or more. As the polyester-based resin, polyethylene terephthalate is preferred from the viewpoint of suppressing the precipitation of crystals.

The range of wavelengths of the rays to be blocked by the package may be from 300 to 335 nm, in consideration of the environment in which the pharmaceutical preparation is generally preserved; however, it is preferably from 300 to 370 nm, and more preferably from 300 to 395 nm, from the viewpoint of improving the stability with respect to light. In particular, in consideration of the use of the pharmaceutical preparation under preservation conditions with a high risk of exposure to wavelengths less than 300 nm, the range of wavelengths of the rays to be blocked by the package is preferably from 270 to 335 nm, more preferably from 270 to 370 nm, and particularly preferably from 270 to 395 nm.

As used herein, the expression "block a ray" in the above-described range of wavelengths means that the average value of transmittance of the rays in the range of wavelengths is 40% or less. Thus, the "package that blocks a ray with a wavelength of 300 to 335 nm", for example, means a package having an average value of transmittance of light with a wavelength of 300 to 335 nm of 40% or less.

Note that from the viewpoint of further improving the stability of the package with respect to light, the average value of transmittance of rays in the above-described range of wavelengths is preferably 30% or less, more preferably 25% or less, still more preferably 20% or less, still more preferably 15% or less, still more preferably 10% or less, and particularly preferably 5% or less.

Note that the average value of transmittance of light in a specific range of wavelengths can be measured herein by measuring light transmittance of the package in air per 0.5 nm in the range of wavelengths, using a spectrophotometer, and then calculating an average value of the measured light transmittances. U-3900 (Hitachi High-Technologies Corporation), for example, may be used as the spectrophotometer.

A specific means for blocking rays in the above-described range of wavelengths may, for example, be a method utilizing a substance that blocks rays in the above-described range of wavelengths, although not particularly limited thereto. More specific methods include:
a method in which a substance that blocks rays in the above-described range of wavelengths is incorporated into the package (for example, a method in which a substance that blocks rays in the above-described range of wavelengths is added into glass or resin, and the mixture is molded in the shape of a container);
a method in which a member (such as a film) containing a substance that blocks rays in the above-described range of wavelengths is incorporated into the surface of the package (at least one surface of the inner and outer sides of the package) (for example, a method in which a substance that blocks rays in the above-described range of wavelengths is added into resin to form a heat-shrinkable film, and the heat-shrinkable film is wound around an outer surface of the container);
a method in which a substance that blocks rays in the above-described range of wavelengths is applied to a surface of the package (at least one surface of the inner and outer sides of the package); and
a method in which a substance that blocks rays in the above-described range of wavelengths is used as a main material of the package (for example, a method in which a container is mainly composed of a metal and cardboard).

The substance that blocks rays in the above-described range of wavelengths is not particularly limited, but is preferably a material that prevents transmission of ultraviolet light, such as an ultraviolet absorber or an ultraviolet scattering agent, in that it can make the inside of the package visible. Specific examples of ultraviolet scattering agents include titanium oxide and zinc oxide. Examples of ultraviolet absorbers include benzotriazole-based ultraviolet absorbers such as 2-(2H-benzotriazol-2-yl)-p-cresol (for example, Tinuvin P: BASF Corporation), 2-(2H-benzotriazol-2-yl)-4,6-bis(1-methyl-1-phenylethyl)phenol (for example, Tinuvin 234: BASF Corporation), 2-(3,5-dit-butyl-2-hydroxyphenyl)benzotriazole (for example, Tinuvin 320: BASF Corporation), 2-[5-chloro(2H)-benzotriazol-2-yl]-4-methyl-6-(tert-butyl)phenol (for example, Tinuvin 326: BASF Corporation), 2-(3,5-di-t-butyl-2-hydroxyphenyl)-5-chlorobenzotriazole (for example, Tinuvin 327: BASF Corporation), 2-(2H-benzotriazol-2-yl)-4,6-di-tert-pentylphenol (for example, Tinuvin PA328: BASF Corporation), 2-(2H-benzotriazol-2-yl)-4-(1,1,3,3-tetramethylbutyl)phenol (for example, Tinuvin 329: BASF Corporation), 2,2'-methylenebis[6-(2H-benzotriazol-2-yl)-4-(1,1,3,3-tetramethylbutyl)phenol (for example, Tinuvin 360: BASF Corporation), a reaction product of methyl 3-(3-(2H-benzotriazol-2-yl)-5-tert-butyl-4-hydroxyphenyl)propionate and polyethylene glycol 300 (for example, Tinuvin 213: BASF Corporation), 2-(2H-benzotriazol-2-yl)-6-dodecyl-4-methylphenol (for example, Tinuvin 571: BASF Corporation), 2-(2'-hydroxy-3',5'-di-t-amylphenyl)benzotriazole, 2-[2'-hydroxy-3'-(3",4",5",6"-tetrahydrophthalimidomethyl)-5'-methylphenyl]benzotriazole, and 2,2'-methylenebis[4-(1,1,3,3-tetramethylbutyl)-6-(2H-benzotriazol-2-yl)phenol]; cyanoacrylate-based ultraviolet absorbers such as 2,2-bis{[2-cyano-3,3-diphenylacryloyloxy]methyl}propane-1,3-diyl=bis(2-cyano-3,3-diphenylacrylate) (for example, Uvinul 3030 FF: BASF Corporation), ethyl 2-cyano-3,3-diphenylacrylate (for example, Uvinul 3035: BASF Corporation), and 2-ethylhexyl 2-cyano-3,3-diphenylacrylate (for example, Uvinul 3039: BASF Corporation); triazine-based ultraviolet absorbers such as 2-(4,6-diphenyl-1,3,5-triazin-2-yl)-5-[(hexyl)oxy]-phenol (for example, Tinuvin 1577 ED: BASF Corporation); benzophenone-based ultraviolet absorbers such as octabenzone (for example, Chimassorb 81: BASF Corporation), 2,2'-dihydroxy-4,4'-dimethoxybenzophenone (for example, Uvinul 3049: BASF Corporation), 2,2'-4,4'-tetrahydrobenzophenone (for example, Uvinul 3050: BASF Corporation), oxybenzone, hydroxymethoxybenzophenonesulfonic acid, sodium hydroxymethoxybenzophenone sulfonate, dihydroxydimethoxybenzophenone, sodium dihydroxydimethoxybenzophenone disulfonate, dihydroxybenzophenone, and tetrahydroxybenzophenone; cinnamate-based ultraviolet absorbers such as methyl diisopropylcinnamate, cinoxate, glyceryl mono-2-ethylhexanoate di-p-methoxycinnamate, isopropyl p-methoxycinnamate-diisopropyl cinnamate ester mixture, 2-ethylhexyl p-methoxycinnamate, and benzyl cinnamate; benzoate-based ultraviolet absorbers such as p-aminobenzoic acid, ethyl p-aminobenzoate, glyceryl p-aminobenzoate, amyl p-dimethylaminobenzoate, 2-ethylhexyl p-dimethylaminobenzoate, and ethyl 4-[N,N-di(2-hydroxypropyl)amino]benzoate; salicylate-based ultraviolet absorbers such as ethylene glycol salicylate, octyl salicylate, dipropylene glycol salicylate, phenyl salicylate, homomenthyl salicylate, and methyl salicylate; guaiazulene; 2-ethylhexyl dimethoxybenzylidene dioxoimidazolidine propionate; 2,4,6-tris[4-(2-ethylhexyloxycarbonyl)anilino]1,3,5-triazine; p-hydroxyanisole; 4-tert-butyl-4'-methoxydibenzoylmethane; phenylbenzimidazole sulfonate; and hexyl 2-(4-diethylamino-2-hydroxybenzoyl)benzoate. As the substance that blocks rays in the above-described range of wavelengths, for example, one of these substances or an appropriate combination of two or more of these substances may be designed to block rays in the above-described range of wavelengths. Titanium oxide and benzotriazole-based ultraviolet absorbers are preferred as the substance that blocks rays in the above-described range of wavelengths.

When the substance that blocks rays in the above-described range of wavelengths is incorporated into the material or the member of the package, the proportion of the substance to be incorporated varies depending on the type of the substance, the material of the package or the separate member, and the like, but it may be from 0.001 to 50 mass%, for example, preferably from 0.002 to 25 mass%, and particularly preferably about from 0.01 to 10 mass%, in the material or the separate member of the package.

Note that as used herein, the "package that blocks a ray" also includes a case where only a portion of the package blocks a ray. In this package, based on its inner surface, the portion of the package that blocks a ray preferably forms 10% or more, more preferably forms 30% or more, still more preferably 50% or more, and particularly preferably 70% or more, of the total area of the inner surface of the package.

The inside of the package is preferably visible (observable) by the naked eye, for example. When the inside is visible, the following advantages are produced. For example, the presence or absence of any foreign matter can be inspected in the manufacturing steps of the pharmaceutical preparation, and the residual amount of the contents (aqueous composition) can be examined by a user of the pharmaceutical preparation.

As used herein, the expression "inside is visible" refers to the state in which the inside of the package is visible at least through a portion of the outer surface of the package (typically, for example, even if the side surface of a container for eye drops having a substantially cylindrical shape cannot be seen through due to the presence of a shrinkable film or the like, it can be determined that the "inside is visible" if the bottom surface of the container is visible.).

Note that for visibility, the package may have transparency of a certain degree or higher; specifically, the package may have an average value of transmittance of light in the visible light region (450 to 750 nm) of about 30% or more, for example (more preferably about 40% or more, and particularly preferably about 50% or more), although not limited thereto.

Specific embodiments of the package are preferably as follows, for example:
1) the primary package is a package (more preferably a package of which inside is visible), which is a container (a container preferably made of plastic, more preferably made of polyolefin-based resin or polyester-based resin (preferably a container for eye drops)) into which a substance that prevents transmission of ultraviolet light (more preferably one or more selected from the group consisting of ultraviolet scattering agents and ultraviolet absorbers, particularly preferably one or more selected from the group consisting of zinc oxide, titanium oxide and benzotriazole-based ultraviolet absorbers) has been mixed;
2) the primary package is a package (more preferably a package of which inside is visible), which is a container (a container preferably made of plastic, more preferably made of polyolefin-based resin or polyester-based resin (preferably a container for eye drops)) in which a member (preferably a heat-shrinkable film (shrinkable film)) into which a substance that prevents transmission of ultraviolet light (more preferably one or more selected from the group consisting of ultraviolet scattering agents and ultraviolet absorbers; and particularly preferably one or more selected from the group consisting of zinc oxide, titanium oxide, and benzotriazole-based ultraviolet absorbers) has been mixed is wound around the side surface;
3) the secondary package is a package (preferably a package of which inside is visible), which is a bag (preferably a bag made of plastic, more preferably made of polyolefin-based resin or polyester-based resin (preferably a bag for eye drop instillation)) into which a substance that prevents transmission of ultraviolet light (preferably one or more selected from the group consisting of ultraviolet scattering agents and ultraviolet absorbers, more preferably one or more selected from the group consisting of zinc oxide, titanium oxide and benzotriazole-based ultraviolet absorbers) has been mixed; and
4) the secondary package is a package, which is a box made of paper for storing a container (preferably a container for eye drops).

The means for storing the aqueous composition into the package is not particularly limited, and the package may be filled with the aqueous composition using a conventional method, in accordance with the form of the package and the like.

The disease targeted by the pharmaceutical preparation is not particularly limited, and may be selected as appropriate depending on the pharmacological action or the like of the compound represented by Formula (1) .

Specifically, the pharmaceutical preparation can be used, for example, as a prophylactic or therapeutic agent for ocular hypertension or glaucoma, based on the Rho kinase inhibitory action or intraocular pressure-lowering action of the compound represented by Formula (1). More specifically, examples of types of glaucoma include primary open-angle glaucoma, normal-tension glaucoma, hypersecretion glaucoma, acute closed-angle glaucoma, chronic closed-angle glaucoma, plateau iris syndrome, combined mechanism glaucoma, steroid-induced glaucoma, capsular glaucoma, pigmentary glaucoma, amyloidassociated glaucoma, neovascular glaucoma, and malignant glaucoma.

Further, as disclosed in JP-B-5557408, the pharmaceutical preparation can be used as a prophylactic or therapeutic agent for ocular fundus diseases (lesions that mainly develop in the retina and/or choroidea; specifically, for example, hypertensive or arteriosclerotic ocular fundus abnormalities, central retinal artery occlusion, retinal vein occlusion such as central retinal vein occlusion or branch retinal vein occlusion, diabetic retinopathy, diabetic macular edema, diabetic maculopathy, Eales disease, Coats disease and other congenital retinal vascular abnormalities, von Hippel disease, pulseless disease, macular diseases (such as central serous chorioretinopathy, cystoid macular edema, age-related macular degeneration, macular hole, myopic macular degeneration, vitreoretinal interface maculopathy, drug-related maculopathy, or heredomacular degeneration), retinal detachment (rhegmatogenous, tractional, exudative, or the like), retinitis pigmentosa, or retinopathy of prematurity). More preferably, the pharmaceutical preparation can be used as a prophylactic or therapeutic agent for diabetic retinopathy, diabetic macular edema, or age-related macular degeneration.

### [Examples]

The present invention will be described next in more detail with reference to examples; however, the invention is in no way limited to these examples.

In the following test examples, ripasudil monohydrochloride dihydrate can be produced in accordance with the method described in WO2006/057397, for example.

Moreover, in the following test examples, measurement of ripasudil and its analogs using HPLC was performed using an ODS column as the column, 0.01 mol/L phosphate buffer and acetonitrile as the mobile phase, and an ultraviolet absorptiometer (wavelength: 280 nm) as the detector.

### [Test Example 1] Evaluation of the photostability of ripasudil

The stability of ripasudil with respect to light was evaluated by examining the presence or absence of degradation products formed by exposure to light.

Specifically, powdery ripasudil monohydrochloride dihydrate was spread in a glass petri dish to a thickness of 3 mm or less, and then irradiated with 4000 lux of light to give a cumulative radiation dose of 400,000, 800,000, or 1,200,000 lux·hr, at 25°C, with a D65 fluorescent lamp as the light source, using a photostability tester (LT-120A: Nagano Science Corporation).

To examine the presence or absence of photodegradation products of ripasudil, the presence or absence of an increase in the amount of ripasudil analogs was evaluated for the sample before and after exposure to light. The amount of analogs was evaluated as the ratio (%) of the peak area of analogs relative to the peak area of ripasudil, using HPLC.

The results are shown in Table 1.

**[Table 1]**

| | Before Exposure to Light | Cumulative Radiation Dose (lux·hr) | | |
|---|---|---|---|---|
| | | 400,000 | 800,000 | 1,200,000 |
| Analogs (%) | 0.11 | 0.14 | 0.16 | 0.16 |

As shown in the results set forth in Table 1, the amount of ripasudil analogs did not substantially increase even though the cumulative radiation dose was increased, and no formation of photodegradation products was observed.

The foregoing test results revealed that the compound represented by Formula (1) typified by ripasudil, a salt thereof, or a solvate of the compound or the salt thereof are extremely stable with respect to light.

### [Test Example 2] Evaluation of the photostability of ripasudil in an aqueous composition

The stability of ripasudil incorporated in an aqueous composition with respect to light was evaluated by examining the presence or absence of degradation products formed by exposure to light, as in Test Example 1.

Specifically, an aqueous composition containing, per 100 mL, 0.4896 g of ripasudil monohydrochloride dihydrate (0.4 g, as the free form of ripasudil), 0.4 g of anhydrous sodium dihydrogen phosphate, 2.136 g of glycerin, 0.002 g of benzalkonium chloride, an appropriate amount of sodium hydroxide (pH 6.0), and sterile purified water (balance) was prepared, and this aqueous composition was stored in a transparent container made of polypropylene. The aqueous composition was then irradiated with 4000 lux of light to give a cumulative radiation dose of 300,000, 600,000, or 1,200,000 lux·hr, at 25°C, with a D65 fluorescent lamp as the light source, using a photostability tester (LT-120A: Nagano Science Corporation).

To examine the presence or absence of photodegradation products of ripasudil, the presence or absence of an increase in the amount of ripasudil analogs was evaluated. The amount of analogs was evaluated as the area percentage (%) relative to the total peak area derived from ripasudil and its analogs, using HPLC.

The results are shown in Table 2.

**[Table 2]**

| | Before Exposure to Light | Cumulative Radiation Dose (lux·hr) | | |
|---|---|---|---|---|
| | | 300,000 | 600,000 | 1,200,000 |
| Analogs (%) | 0.17 | 1.29 | 1.95 | 3.67 |

As shown in the results set forth in Table 2, it was revealed that the amount of analogs increases in proportion to an increase in the cumulative radiation dose, and degradation products due to exposure to light are formed. Note that the D65 fluorescent lamp used as the light source emits rays with wavelengths of 300 nm or more.

The photodegradation reaction of an organic compound is dependent on the strength of a specific bond in the compound, and hence, is dependent on the structure of the compound. Thus, the stability of the organic compound with respect to light (susceptibility to photodegradation) was expected to generally have a similar tendency, independent of the state (such as a solid or a liquid) of the organic compound. Unexpectedly, however, the results of Test Examples 1 and 2 revealed that although the compound represented by Formula (1) typified by ripasudil or a salt thereof, or a solvate of the compound or the salt thereof per se is stable with respect to light, it has the property of becoming unstable with respect to light once it is incorporated into an aqueous composition.

### [Test Example 3] Identification of the wavelengths of rays causing the degradation of ripasudil in an aqueous composition No. 1

The wavelengths of rays causing the photodegradation of ripasudil in the aqueous composition examined in Test Example 2 were identified by examining the presence or absence of degradation products formed by exposure to light, by irradiation of rays with a specific range of wavelengths.

Specifically, an aqueous composition containing, per 100 mL, 0.0612 g of ripasudil monohydrochloride dihydrate (0.05 g as the free form of ripasudil), 1.19 g of boric acid, 0.42 g of potassium chloride, 0.002 g of benzalkonium chloride, an appropriate amount of sodium hydroxide (pH 6.7), and sterile purified water (balance) was prepared, and this aqueous composition was stored in a transparent container made of polypropylene. The aqueous composition was then irradiated with rays with wavelengths in the range of approximately 270 to 335 nm, 320 to 395 nm, 370 to 435 nm, 430 to 470 nm, 470 to 530 nm, 525 to 575 nm, 570 to 630 nm, 625 to 675 nm, or 660 to 740 nm, at 25°C, using a spectroradiometer equipped with optical filters (spectral unit: HSU-100S, light source: MAX-302FBD; both from Asahi Spectra Co., Ltd.). Note that the rays of wavelengths in each of the ranges were emitted at a radiation energy of about 15 W·h/m².

To examine the presence or absence of photodegradation products of ripasudil, the presence or absence of an increase in the amount of ripasudil analogs was evaluated, using the same method as that of Test Example 1.

The results are shown in Table 3.

**[Table 3]**

| | Emitted Wavelengths (nm) | | | | | | | | |
|---|---|---|---|---|---|---|---|---|---|
| | 270 to 335 | 320 to 395 | 370 to 435 | 430 to 470 | 470 to 530 | 525 to 575 | 570 to 630 | 625 to 675 | 660 to 740 |
| Analogs (%) | 4.22 | 1.23 | 0.05 | 0.05 | 0.05 | 0.05 | 0.05 | 0.05 | 0.05 |

As shown in the results set forth in Table 3, an increase in the amount of analogs was not observed when rays with wavelengths of 370 nm or more were emitted, and a significant increase in the amount of analogs was observed when rays in the range of shorter wavelengths, particularly rays with wavelengths of 270 to 335 nm, were emitted.

Note that photostability testing exists as one of the tests for examining the preservation stability of pharmaceuticals, and the guidelines of this test prescribe that a D65 light source should be used as the light source. The D65 light source is the internationally recognized standard for outdoor daylight as defined in ISO10977 (1993), and is defined by the values of a spectral distribution in the range of wavelengths of 300 to 830 nm. Based on the above, under preservation conditions generally expected for pharmaceuticals, it is believed that it suffices to take into account light with wavelengths of 300 nm or more.

The foregoing concluded that it is important to block rays with wavelengths of particularly 300 to 335 nm, in order to ensure the photostability of the aqueous composition containing the compound represented by Formula (1) typified by ripasudil or a salt thereof, or a solvate of the compound or the salt thereof.

### [Test Example 4] Identification of the wavelengths of rays causing the degradation of ripasudil in aqueous compositions No. 2

The test was performed as in Test Example 3, except that the aqueous composition was changed to the two types of aqueous compositions shown below, and the wavelengths of emitted rays were changed to 270 to 335 nm, 320 to 395 nm, or 370 to 435 nm.

### <Aqueous Composition A>

An aqueous composition containing, per 100 mL, 0.0612 g of ripasudil monohydrochloride dihydrate (0.05 g, as the free form of ripasudil), 0.4 g of anhydrous sodium dihydrogen phosphate, 0.78 g of potassium chloride, 0.002 g of benzalkonium chloride, an appropriate amount of sodium hydroxide (pH 6.7), and sterile purified water (balance) was prepared and used as Aqueous Composition A.

### <Aqueous Composition B>

An aqueous composition containing, per 100 mL, 0.0612 g of ripasudil monohydrochloride dihydrate (0.05 g, as the free form of ripasudil), 0.98 g of potassium chloride, 0.002 g of benzalkonium chloride, an appropriate amount of sodium hydroxide (pH 6.7), and sterile purified water (balance) was prepared and used as Aqueous Composition B.

The results are shown in Table 4.

**[Table 4]**

| | | Emitted Wavelengths (nm) | | |
|---|---|---|---|---|
| | | 270 to 335 | 320 to 395 | 370 to 435 |
| Analogs (%) | Aqueous Composition A | 3.95 | 1.07 | 0.05 |
| | Aqueous Composition B | 3.47 | 1.02 | 0.05 |

As shown in the results set forth in Table 4, regardless of the compositional differences between the aqueous compositions, an increase in the amount of analogs was not observed when rays with wavelengths of 370 nm or more were emitted, and a significant increase in the amount of analogs was observed when rays in the range of shorter wavelengths, particularly rays with wavelengths of 270 to 335 nm, were emitted.

The foregoing test results of Test Examples 3 and 4 concluded that it is important to block rays with wavelengths of particularly 300 to 335 nm, regardless of the composition, in order to ensure the photostability of the aqueous composition containing the compound represented by Formula (1) typified by ripasudil or a salt thereof, or a solvate of the compound or the salt thereof.

### [Test Example 5] Confirmation of the stabilization of ripasudil in an aqueous composition stored in a plastic container (primary package) containing an ultraviolet absorber

Based on the results obtained in Test Examples 3 and 4, stabilization of an aqueous composition containing ripasudil was attempted by storing the aqueous composition in a container containing an ultraviolet absorber.

Specifically, an aqueous composition containing, per 100 mL, 0.4896 g of ripasudil monohydrochloride dihydrate (0.4 g, as the free form of ripasudil), 0.4 g of anhydrous sodium dihydrogen phosphate, 2.136 g of glycerin, 0.002 g of benzalkonium chloride, an appropriate amount of sodium hydroxide (pH 6.0), and sterile purified water (balance) was prepared. This aqueous composition was then stored in the below-described container for eye drops made of polypropylene, which contained an ultraviolet absorber. The aqueous composition was then irradiated with 4000 lux of light to give a cumulative radiation dose of 1,200,000 lux·hr, at 25°C, with a D65 fluorescent lamp as the light source, using a photostability tester (LT-120A: Nagano Science Corporation).

The amount of ripasudil analogs was evaluated for the sample before and after exposure to light, as in Test Example 1.

The results are shown in Table 5.

### <Plastic container containing an ultraviolet absorber>

A container for eye drops made of polypropylene into which a benzotriazole-based ultraviolet absorber was mixed was used. The container had an average value of transmittance of light in the range of 300 to 335 nm of 7.2% (note that the average value was 6.7% in the range of 300 to 370 nm; 11.7% in the range of 270 to 335; 9.8% in the range of 270 to 370 nm; 10.2% in the range of 300 to 395 nm; and 11.8% in the range of 270 to 395 nm).

Apparently, the container was substantially transparent, and its contents were visible (for example, the transmittance of light in the visible light region (450 to 750 nm) exceeded 65% at all the wavelengths, and the average value thereof was 76.2%).

Note that the light transmittance was measured per 0.5 nm using a spectrophotometer (U-3900: Hitachi High-Technologies), by cutting the container into plate-like pieces, and then setting them on a light path such that light was incident substantially perpendicularly. The average value of light transmittance was calculated as the average value of light transmittances measured per 0.5 nm in a predetermined range of wavelengths.

**[Table 5]**

| | Characteristics of Container | Before Exposure to Light | Cumulative Radiation Dose (lux·hr) |
|---|---|---|---|
| | | | 1,200,000 |
| Analogs (%) | Polypropylene Container Containing Ultraviolet Absorber Average Value of Transmittance of Light at 300 to 335 nm: 7.2% | 0.11 | 0.55 |

The results set forth in Table 5 revealed that the photostability of ripasudil in the aqueous composition is improved by storing the aqueous composition containing ripasudil in a container having an average value of transmittance of light in the range of 300 to 335 nm of 7.2% (note that the average value was 6.7% in the range of 300 to 370 nm; 11.7% in the range of 270 to 335 nm; 9.8% in the range of 270 to 370 nm; 10.2% in the range of 300 to 395 nm; and 11.8% in the range of 270 to 395 nm).

### [Test Example 6] Confirmation of the stabilization of ripasudil in the aqueous composition stored in a container (primary package) including a shrinkable film containing an ultraviolet scattering agent

Stabilization of the aqueous composition containing ripasudil was attempted by storing the aqueous composition in a container containing an ultraviolet scattering agent, as in Test Example 5.

Specifically, the test was performed as in Test Example 5, except that the below-described container including a shrinkable film containing an ultraviolet scattering agent was used instead of the container containing an ultraviolet absorber, and a white fluorescent lamp (one used in a hospital dispensary and the like) was used as the light source instead of the D65 fluorescent lamp.

The results are shown in Table 6.

### <Container including a shrinkable film containing an ultraviolet scattering agent>

A white shrinkable film (heat-shrinkable film) (Iwata Label Co., Ltd.) into which 40 to 45 mass% of titanium oxide was mixed as an ultraviolet scattering agent was wound around a side surface of a general container for eye drops made of polypropylene not containing an ultraviolet absorber, an ultraviolet scattering agent, or the like, in accordance with a conventional method, and the resulting container was used. The shrinkable film containing titanium oxide had an average value of transmittance of light in the range of 300 to 335 nm of 0.3% (note that the average value was 0.4% in the range of 300 to 370 nm; 0.1% in the range of 270 to 335 nm; 0.3% in the range of 270 to 370 nm; 0.4% in the range of 300 to 395 nm; and 0.3% in the range of 270 to 395 nm).

Since the shrinkable film was not transparent, the contents were not visible through the side surface of the container in this state (for example, the average value of transmittance of light in the visible light region (450 to 750 nm) was 1.1%). Thus, slits with about a width of 5 mm were provided in the lower half of the side surface of the container to make the amount of the contents visible (this portion was not able to block the rays). The contents were visible through the bottom surface of the container, since the shrinkable film was not wound around the bottom surface.

Note that the light transmittance was measured per 0.5 nm using a spectrophotometer (U-3900: Hitachi High-Technologies). The average value of light transmittance was calculated as the average value of light transmittances measured per 0.5 nm in a predetermined range of wavelengths.

**[Table 6]**

| | Characteristics of Container | Before Exposure to Light | Cumulative Radiation Dose (lux·hr) |
|---|---|---|---|
| | | | 1,200,000 |
| Analogs (%) | Polypropylene Container Wound with the Shrinkable Film Containing Titanium Oxide Average Value of Transmittance of Light at 300 to 335 nm: 0.3% | 0.13 | 0.28 |

The results set forth in Table 6 revealed that the photostability of ripasudil in the aqueous composition is improved by storing the aqueous composition containing ripasudil in a container having an average value of transmittance of light in the range of 300 to 335 nm of 0.3% (note that the average value was 0.4% in the range of 300 to 370 nm; 0.1% in the range of 270 to 335 nm; 0.3% in the range of 270 to 370 nm; 0.4% in the range of 300 to 395 nm; and 0.3% in the range of 270 to 395 nm).

The results of Test Examples 5 and 6 confirmed that the photostability of the compound represented by Formula (1) in the aqueous composition is improved by storing the aqueous composition containing the compound represented by Formula (1) typified by ripasudil or a salt thereof, or a solvate of the compound or the salt thereof in the package that blocks rays with wavelengths of 300 to 335 nm.

Note that similar results can also be obtained when a transparent bag for eye drop instillation (Seisannipponsha Ltd.) made of polyethylene containing an ultraviolet absorber is used as the secondary package, instead of the containers used in Test Examples 5 and 6.

Note that the bag for eye drop instillation had an average value of transmittance of light in the range of 300 to 335 nm of 0.4%; 1.4% in the range of 300 to 370 nm; 0.2% in the range of 270 to 335 nm; 0.9% in the range of 270 to 370 nm; 3.6% in the range of 300 to 395 nm; and 2.7% in the range of 270 to 395 nm.

Furthermore, the container was apparently white and semi-transparent, and its contents were visible (for example, the transmittance of light in the visible light region (450 to 750 nm) exceeded 45% at all the wavelengths, and the average value thereof was 70.6%).

### [Test Example 7] Preservation test No. 1

An aqueous composition of the formulation shown in Table 7 was prepared in accordance with a conventional method, and then placed in a container made of polyethylene (PE), polypropylene (PP), or polyvinyl chloride (PVC) to produce a pharmaceutical preparation.

Each of the resulting pharmaceutical preparations was preserved at 60°C for 3 months, and a color difference (ΔYI) before and after the preservation was measured using a color difference meter (spectrophotometer, CM-700d: Konica Minolta Sensing, Inc.). A ΔYI value of 5 or more was rated as "b", and a Δ YI value of less than 5 was rated as "a".

The results are shown in Table 8.

**[Table 7]**

| Components | Quantity (per 100 mL) |
|---|---|
| Ripasudil Monohydrochloride Dihydrate | 0.4896 g (0.4 g as the free form) |
| Anhydrous Sodium Dihydrogen Phosphate | 0.8 g |
| Sodium Hydroxide | q.s. (pH6.7) |
| Purified Water | Balance |

**[Table 8]**

| Material of Container | ΔYI | Evaluation |
|---|---|---|
| PE | 4.07 | a |
| PP | 4.42 | a |
| PVC | 7.45 | b |

As shown in the results set forth in Table 8, when the aqueous composition containing ripasudil was stored in the container made of polyolefin-based resin, such as polyethylene (PE) or polypropylene (PP), the ΔYI value was reduced even after the aqueous composition was preserved at high temperature for a long period of time; in contrast, the ΔYI value was increased when the aqueous composition was stored in the container made of polyvinyl chloride (PVC).

### [Test Example 8] Preservation test No. 2

An aqueous composition of the formulation shown in Table 9 was prepared in accordance with a conventional method, and then placed in a container made of polyethylene (PE) or polypropylene (PP) to produce a pharmaceutical preparation.

Each of the resulting pharmaceutical preparations was preserved at 60°C for 3 months, and a color difference (ΔYI) before and after the preservation was measured using a color difference meter (spectrophotometer, CM-700d: Konica Minolta Sensing, Inc.). A ΔYI value of 5 or more was rated as "b", and a Δ YI value of less than 5 was rated as "a".

The results are shown in Table 10.

**[Table 9]**

| Components | Quantity (per 100 mL) |
|---|---|
| Ripasudil Monohydrochloride Dihydrate | 0.4896 g(0.4 g as the free form) |
| Boric Acid | 1.2 g |
| Sodium Hydroxide | q.s. (pH6.7) |
| Purified Water | Balance |

**[Table 10]**

| Material of Container | ΔYI | Evaluation |
|---|---|---|
| PE | 4.15 | a |
| PP | 4.64 | a |

As shown in the results set forth in Table 10, even though the formulation of the aqueous composition was changed, when the aqueous composition was stored in the container made of polyolefin-based resin, such as polyethylene (PE) or polypropylene (PP), the ΔYI value was reduced even after preservation at high temperature for a long period of time.

The foregoing results of Test Examples 7 and 8 revealed that when the aqueous composition containing the compound represented by Formula (1) typified by ripasudil or a salt thereof, or a solvate of the compound or the salt thereof is stored in a container made of polyolefin-based resin, ΔYI is reduced, and excellent stability can be achieved.

### [Test Example 9] Preservation test No. 3

An aqueous composition of the formulation shown in Table 11 was prepared in accordance with a conventional method, and then placed in a container made of polyethylene terephthalate (PET) or polyvinyl chloride (PVC) to produce a pharmaceutical preparation.

Each of the resulting pharmaceutical preparations was preserved at 0°C for 2 days, and then visually evaluated for the presence or absence of precipitation of crystals. Note that the absence of precipitation of crystals was rated as "a", and the presence of precipitation of crystals was rated as "b".

The results are shown in Table 12.

**[Table 11]**

| Components | Quantity (per 100 mL) |
|---|---|
| Ripasudil Monohydrochloride Dihydrate | 0.4896 g(0.4 g as the free form) |
| Anhydrous Sodium Dihydrogen Phosphate | 0.6 g |
| Sodium Hydroxide | q.s. (pH6.7) |
| Purified Water | Balance |

**[Table 12]**

| Material of Container | Presence or Absence of Precipitation of Crystals |
|---|---|
| PET | a |
| PVC | b |

As shown in the results set forth in Table 12, when the aqueous composition containing ripasudil was stored in the container made of polyester-based resin such as polyethylene terephthalate (PET), no precipitation of crystals was observed even after it was preserved at low temperature; in contrast, when the aqueous composition was stored in the container made of polyvinyl chloride (PVC), precipitation of crystals was observed.

### [Test Example 10] Preservation test No. 4

An aqueous composition of the formulation shown in Table 13 was prepared in accordance with a conventional method, and then placed in a container made of polyethylene terephthalate (PET) to produce a pharmaceutical preparation.

The resulting pharmaceutical preparation was preserved at 0°C for 21 days, and then visually evaluated for the presence or absence of precipitation of crystals. Note that the absence of precipitation of crystals was rated as "a", and the presence of precipitation of crystals was rated as "b".

The results are shown in Table 14.

**[Table 13]**

| Components | Quantity (per 100 mL) |
|---|---|
| Ripasudil Monohydrochloride Dihydrate | 0.4896 g(0.4 g as the free form) |
| Boric Acid | 1.2 g |
| Sodium Hydroxide | q.s. (pH6.7) |
| Purified Water | Balance |

**[Table 14]**

| Material of Container | Presence or Absence of Precipitation of Crystals |
|---|---|
| PET | a |

As shown in the results set forth in Table 14, even though the formulation of the aqueous composition was changed, when the aqueous composition was stored in the container made of polyester-based resin such as polyethylene terephthalate (PET), no precipitation of crystals was observed even after preservation at low temperature.

The foregoing results of Test Examples 9 and 10 revealed that when the aqueous composition containing the compound represented by Formula (1) typified by ripasudil or a salt thereof, or a solvate of the compound or the salt thereof was stored in a container made of polyester-based resin, precipitation of crystals is relatively unlikely to occur even after preservation at low temperature, and excellent preservation stability can be achieved.

### [Production Examples 1 to 27]

Pharmaceutical preparations of Production Examples 1 to 27 can be produced by preparing aqueous compositions containing the components in the quantities (amounts (g) per 100 mL of the aqueous composition) shown in Tables 15 to 17 in accordance with a conventional method, and storing them in the container (primary package) for eye drops made of polypropylene into which a benzotriazole-based ultraviolet absorber has been mixed, which was used in Test Example 5.

**[Table 15]**

| | Productio n Example 1 | Productio n Example 2 | Productio n Example 3 | Productio n Example 4 | Productio n Example 5 | Productio n Example 6 | Productio n Example 7 | Productio n Example 8 | Productio n Example 9 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 0.8 | 0.8 |
| Sodium Chloride | 0.65 | | | | 0.3 | 0.3 | 0.3 | 0.3 | |
| Glycerin | | 2 | | | 1 | | | 0.5 | 1 |
| Propylene Glycol | | | 2 | | | 1 | | 0.5 | 1 |
| Potassium Chloride | | | | 0.6 | | | 0.3 | | |
| Boric Acid | | | | | | | | | |
| Borax | | | | | | | | | |
| Sodium Dihydrogenphospha te Monohydrate | 0.4 | 0.4 | 0.4 | | | 0.4 | 0.4 | 0.4 | 0.4 |
| Dibasic Sodium Phosphate Hydrate | | | | | | | | q.s. | q.s. |
| Anhydrous Sodium Monohydrogen Phosphate | | | | | | q.s. | q.s. | | |
| Potassium Dihydrogenphospha te | | | | 0.4 | 0.4 | | | | |
| Sodium Hydroxide | q.s. | q.s. | q.s. | q.s. | q.s. | | | | |
| Trometamol | | | | | | | | | |
| Hydrochloric Acid | | | | | | | | | |
| Citric Acid Hydrate | 0.1 | | | | | 0.1 | | | |
| Sodium Acetate Hydrate | | 0.1 | | | | 0.1 | | | |
| Sodium Edetate | | | | 0.1 | | | 0.1 | | |
| Benzalkonium Chloride | 0.001 | 0.005 | | 0.001 | 0.005 | 0.01 | 0.001 | 0.005 | |
| Benzethonium Chloride | | | | | | | | | 0.01 |
| Methyl Parahydroxybenzoa te | | | 0.01 | | | | 0.01 | | |
| Propyl Parahydroxybenzoa te | | | 0.01 | | | | 0.01 | | |
| Chlorobutanol | | | | 0.2 | | | | 0.2 | |
| Polysorbate 80 | 0.3 | | | 0.3 | 0.3 | | | 0.3 | 0.3 |
| Polyoxyethylene Castor Oil 60 | | 0.3 | | 0.3 | | 0.3 | | 0.3 | 0.3 |
| Polyethylene Glycol Monostearate | | | 1.5 | 1.5 | | | 1.5 | | 1.5 |
| Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
| pH | 5 | 5 | 6 | 6 | 6.5 | 6.5 | 7 | 7 | 8 |

**[Table 16]**

| | Productio n Example 10 | Productio n Example 11 | Productio n Example 12 | Productio n Example 13 | Productio n Example 14 | Productio n Example 15 | Productio n Example 16 | Productio n Example 17 | Productio n Example 18 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 0.8 | 0.8 |
| Sodium Chloride | 0.65 | | | | 0.3 | 0.3 | 0.3 | 0.3 | |
| Glycerin | | 2 | | | 1 | | | 0.5 | 1 |
| Propylene Glycol | | | 2 | | | 1 | | 0.5 | 1 |
| Potassium Chloride | | | | 0.6 | | | 0.3 | | |
| Boric Acid | 1.0 | 1.0 | 1.0 | 1.0 | 1.0 | 1.0 | 1.0 | 1.0 | 1.0 |
| Borax | | | | | q.s. | q.s. | q.s. | q.s. | q.s. |
| Sodium Dihydrogenphospha te Monohydrate | | | | | | | | | |
| Dibasic Sodium Phosphate Hydrate | | | | | | | | | |
| Anhydrous Sodium Monohydrogen Phosphate | | | | | | | | | |
| Potassium Dihydrogen phospha te | | | | | | | | | |
| Sodium Hydroxide | q.s. | q.s. | q.s. | q.s. | | | | | |
| Trometamol | | | | | | | | | |
| Hydrochloric Acid | | | | | | | | | |
| Citric Acid Hydrate | 0.1 | | | | | 0.1 | | | |
| Sodium Acetate Hydrate | | 0.1 | | | | 0.1 | | | |
| Sodium Edetate | | | | 0.1 | | | 0.1 | | |
| Benzalkonium Chloride | 0.001 | 0.005 | | 0.001 | 0.005 | 0.01 | 0.001 | 0.005 | |
| Benzethonium Chloride | | | | | | | | | 0.01 |
| Methyl Parahydroxybenzoa te | | | 0.01 | | | | 0.01 | | |
| Propyl Parahydroxybenzoa te | | | 0.01 | | | | 0.01 | | |
| Chlorobutanol | | | | 0.2 | | | | 0.2 | |
| Polysorbate 80 | 0.3 | | | 0.3 | 0.3 | | | 0.3 | 0.3 |
| Polyoxyethylene Castor Oil 60 | | 0.3 | | 0.3 | | 0.3 | | 0.3 | 0.3 |
| Polyethylene Glycol Monostearate | | | 1.5 | 1.5 | | | 1.5 | | 1.5 |
| Purified Water | Total Amount 100 mL | Total Amount 100 mL | Total Amount 100 mL | Total Amount 100 mL | Total Amount 100 mL | Total Amount 100 mL | Total Amount 100 mL | Total Amount 100 mL | Total Amount 100 mL |
| pH | 5 | 5 | 6 | 6 | 6.5 | 6.5 | 7 | 7 | 8 |

**[Table 17]**

| | Productio n Example 19 | Productio n Example 20 | Productio n Example 21 | Productio n Example 22 | Productio n Example 23 | Productio n Example 24 | Productio n Example 25 | Productio n Example 26 | Productio n Example 27 |
|---|---|---|---|---|---|---|---|---|---|
| Ripasudil Monohydrochloride Dihydrate (as the amount of the free form) | 0.2 | 0.2 | 0.2 | 0.4 | 0.4 | 0.4 | 0.8 | 0.8 | 0.8 |
| Sodium Chloride | 0.65 | | | | 0.3 | 0.3 | 0.3 | 0.3 | |
| Glycerin | | 2 | | | 1 | | | 0.5 | 1 |
| Propylene Glycol | | | 2 | | | 1 | | 0.5 | 1 |
| Potassium Chloride | | | | 0.6 | | | 0.3 | | |
| Boric Acid | | | | | | | | | |
| Borax | | | | | | | | | |
| Sodium Dihydrogen phospha te Monohydrate | | | | | | | | | |
| Dibasic Sodium Phosphate Hydrate | | | | | | | | | |
| Anhydrous Sodium Monohydrogen Phosphate | | | | | | | | | |
| Potassium Dihydrogenphospha te | | | | | | | | | |
| Sodium Hydroxide | | | | | | | | | |
| Trometamol | 1.5 | 1.5 | 1.5 | 1.5 | 1.5 | 1.5 | 1.5 | 1.5 | 1.5 |
| Hydrochloric Acid | q.s. | q.s. | q.s. | q.s. | q.s. | q.s. | q.s. | q.s. | q.s. |
| Citric Acid Hydrate | 0.1 | | | | | 0.1 | | | |
| Sodium Acetate Hydrate | | 0.1 | | | | 0.1 | | | |
| Sodium Edetate | | | | 0.1 | | | 0.1 | | |
| Benzalkonium Chloride | 0.001 | 0.005 | | 0.001 | 0.005 | 0.01 | 0.001 | 0.005 | |
| Benzethonium Chloride | | | | | | | | | 0.01 |
| Methyl Parahydroxybenzoa te | | | 0.01 | | | | 0.01 | | |
| Propyl Parahydroxybenzoa te | | | 0.01 | | | | 0.01 | | |
| Chlorobutanol | | | | 0.2 | | | | 0.2 | |
| Polysorbate 80 | 0.3 | | | 0.3 | 0.3 | | | 0.3 | 0.3 |
| Polyoxyethylene Castor Oil 60 | | 0.3 | | 0.3 | | 0.3 | | 0.3 | 0.3 |
| Polyethylene Glycol Monostearate | | | 1.5 | 1.5 | | | 1.5 | | 1.5 |
| Purified Water | Total Amount | Total Amount | Total Amount | Total Amount | Total Amount | Total Amount | Total Amount | Total Amount | Total Amount |

| | 100 mL | 100 mL | 100 mL | 100 mL | 100 mL | 100 mL | 100 mL | 100 mL | 100 mL |
|---|---|---|---|---|---|---|---|---|---|
| pH | 5 | 5 | 6 | 6 | 6.5 | 6.5 | 7 | 7 | 8 |

### [Production Examples 28 to 54]

Pharmaceutical preparations of Production Examples 28 to 54 can be produced as in Production Examples 1 to 27, using the same container as that used in Test Example 5, except that the container is made of polyethylene instead of polypropylene.

### [Production Examples 55 to 81]

Pharmaceutical preparations of Production Examples 55 to 81 can be produced as in Production Examples 1 to 27, except that the container (primary package) for eye drops made of polypropylene in which the shrinkable film containing titanium oxide is wound around the side surface, which was used in Test Example 6, is used instead of the container for eye drops made of polypropylene into which a benzotriazole-based ultraviolet absorber has been mixed.

### [Production Examples 82 to 108]

Pharmaceutical preparations of Production Examples 82 to 108 can be produced as in Production Examples 1 to 27, except that a container for eye drops made of polyethylene (one that does not block rays in the range of wavelengths of 300 to 335 nm) is used instead of the container for eye drops made of polypropylene into which a benzotriazole-based ultraviolet absorber has been mixed, and then the pharmaceutical preparations are placed in the above-described transparent bag for eye drop instillation (Seisannipponsha Ltd.) made of polyethylene containing an ultraviolet absorber (secondary package).

### [Production Examples 109 to 135]

Pharmaceutical preparations of Production Examples 109 to 135 can be produced as in Production Examples 1 to 27, except that a container for eye drops made of polyethylene terephthalate (one that does not block rays in the range of wavelengths of 300 to 335 nm) is used instead of the container for eye drops made of polypropylene into which a benzotriazole-based ultraviolet absorber has been mixed, and then the pharmaceutical preparations are placed in boxes made of paper (secondary package).

### [Production Examples 136 to 270]

Pharmaceutical preparations of Production Examples 136 to 270 can be produced in accordance with a conventional method as in Production Examples 1 to 135, except that instead of ripasudil monohydrochloride dihydrate, an equal amount of 4-bromo-5-[[(2S)-2-methyl-1,4-diazepan-1-yl]sulfonyl]isoquinoline is used.

### [Industrial Applicability]

In accordance with the present invention, pharmaceutical preparations having excellent stability can be provided, which can be advantageously used in the pharmaceutical industry, for example.

## Claims

1. A pharmaceutical preparation obtained by storing an aqueous composition comprising a compound represented by Formula (1): 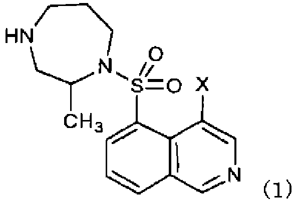
wherein X represents a halogen atom,
or a salt thereof, or a solvate of the compound or the salt thereof, in a package that blocks a ray with a wavelength of 300 to 335 nm.

2. The pharmaceutical preparation according to claim 1, wherein the inside of the package is visible.

3. The pharmaceutical preparation according to claim 1 or 2, wherein a primary package is a container comprising a substance that prevents transmission of ultraviolet light.

4. The pharmaceutical preparation according to any one of claims 1 to 3, wherein the primary package is a container comprising a member comprising a substance that prevents transmission of ultraviolet light.

5. The pharmaceutical preparation according to any one of claims 1 to 4, wherein the primary package is a container made of polyolefin-based resin or polyester-based resin.

6. The pharmaceutical preparation according to any one of claims 1 to 5, which comprises, as a secondary package, a bag comprising a substance that prevents transmission of ultraviolet light.

7. An aqueous composition comprising a compound represented by Formula (1): 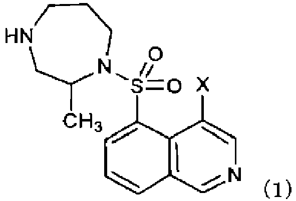
wherein X represents a halogen atom,
or a salt thereof, or a solvate of the compound or the salt thereof,
the aqueous composition being stored in a package that blocks a ray with a wavelength of 300 to 335 nm.

8. The aqueous composition according to claim 7, wherein the inside of the package is visible.

9. The aqueous composition according to claim 7 or 8, wherein a primary package is a container comprising a substance that prevents transmission of ultraviolet light.

10. The aqueous composition according to any one of claims 7 to 9, wherein the primary package is a container comprising a member comprising a substance that prevents transmission of ultraviolet light.

11. The aqueous composition according to any one of claims 7 to 10, wherein the primary package is a container made of polyolefin-based resin or polyester-based resin.

12. The aqueous composition according to any one of claims 7 to 11, which comprises, as a secondary package, a bag comprising a substance that prevents transmission of ultraviolet light.

13. A method for improving photostability of a compound represented by Formula (1): 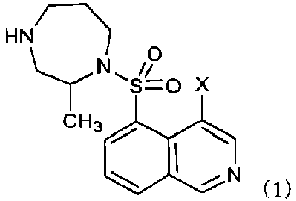
wherein X represents a halogen atom,
or a salt thereof, or a solvate of the compound or the salt thereof, in an aqueous composition,
the method comprising the step of storing the aqueous composition comprising the compound represented by Formula (1), or a salt thereof, or a solvate of the compound or the salt thereof, in a package that blocks a ray with a wavelength of 300 to 335 nm.

14. A method for preserving a compound represented by Formula (1): 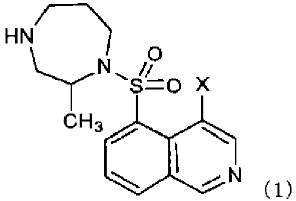
wherein X represents a halogen atom,
or a salt thereof, or a solvate of the compound or the salt thereof,
the method comprising the step of storing an aqueous composition comprising the compound represented by Formula (1), or a salt thereof, or a solvate of the compound or the salt thereof, in a package that blocks a ray with a wavelength of 300 to 335 nm.

15. The pharmaceutical preparation according to claim 1, the aqueous composition according to claim 7, the method according to claim 13, or the method according to claim 14, wherein the compound represented by Formula (1) is ripasudil.

## Patentansprüche

1. Pharmazeutische Zubereitung, die durch Aufbewahren einer wässrigen Zusammensetzung erhalten wird, die eine Verbindung der Formel (1) umfasst: 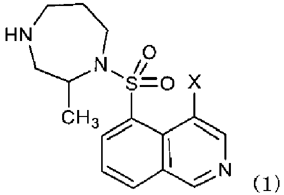 wobei X für ein Halogenatom steht oder ein Salz derselben oder ein Solvat der Verbindung oder ein Salz desselben, in einer Packung, die Strahlung mit einer Wellenlänge von 300 bis 335 nm blockiert.

2. Pharmazeutische Zubereitung nach Anspruch 1, wobei das Innere der Verpackung sichtbar ist.

3. Pharmazeutische Zubereitung nach Anspruch 1 oder 2, wobei die Primärverpackung ein Behälter ist, der eine Substanz umfasst, die die Transmission von ultraviolettem Licht verhindert.

4. Pharmazeutische Zubereitung nach einem der Ansprüche 1 bis 3, wobei die Primärverpackung ein Behälter ist, der ein Element umfasst, das eine Substanz umfasst, die die Transmission von ultraviolettem Licht verhindert.

5. Pharmazeutische Zubereitung nach einem der Ansprüche 1 bis 4, wobei die Primärverpackung ein Behälter aus einem Harz auf Polyolefinbasis oder einem Harz auf Polyesterbasis ist.

6. Pharmazeutische Zubereitung nach einem der Ansprüche 1 bis 5, die als Sekundärverpackung einen Beutel umfasst, der eine Substanz umfasst, die die Transmission von ultraviolettem Licht verhindert.

7. Wässrige Zusammensetzung, umfassend eine Verbindung der Formel (1): 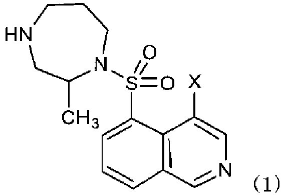 wobei X für ein Halogenatom steht oder ein Salz derselben oder ein Solvat der Verbindung oder ein Salz desselben, wobei die wässrige Zusammensetzung in einer Verpackung aufbewahrt wird, die Strahlen mit einer Wellenlänge von 300 bis 335 nm blockiert.

8. Wässrige Zusammensetzung nach Anspruch 7, wobei das Innere der Verpackung sichtbar ist.

9. Wässrige Zusammensetzung nach Anspruch 7 oder 8, wobei die Primärverpackung ein Behälter ist, der eine Substanz umfasst, die die Transmission von ultraviolettem Licht verhindert.

10. Wässrige Zusammensetzung nach einem der Ansprüche 7 bis 9, wobei die Primärverpackung ein Behälter ist, der ein Element umfasst, das eine Substanz umfasst, die die Transmission von ultraviolettem Licht verhindert.

11. Wässrige Zusammensetzung nach einem der Ansprüche 7 bis 10, wobei die Primärverpackung ein Behälter aus einem Harz auf Polyolefinbasis oder einem Harz auf Polyesterbasis ist.

12. Wässrige Zusammensetzung nach einem der Ansprüche 7 bis 11, die als Sekundärverpackung einen Beutel umfasst, der eine Substanz umfasst, die die Transmission von ultraviolettem Licht verhindert.

13. Verfahren zur Verbesserung der Photostabilität einer Verbindung der Formel (1): 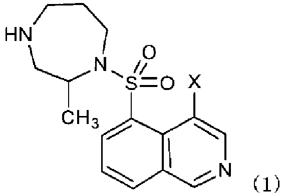 wobei X für ein Halogenatom steht oder eines Salzes derselben oder eines Solvats der Verbindung oder eines Salz desselben, in einer wässrigen Zusammensetzung, wobei das Verfahren einen Schritt des Aufbewahrens der wässrigen Zusammensetzung, die die Verbindung der Formel (1) umfasst, oder ein Salz derselben oder ein Solvat der Verbindung oder ein Salz desselben, in einer Packung umfasst, die Strahlung mit einer Wellenlänge von 300 bis 335 nm blockiert.

14. Verfahren zum Konservieren einer Verbindung der Formel (1): 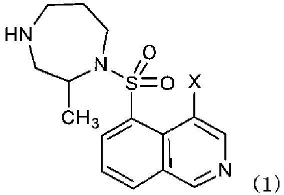 wobei X für ein Halogenatom steht oder eines Salzes derselben oder eines Solvats der Verbindung oder eines Salzes desselben, wobei das Verfahren einen Schritt des Aufbewahrens einer wässrigen Zusammensetzung, die die Verbindung der Formel (1) umfasst, oder ein Salz derselben oder ein Solvat der Verbindung oder ein Salz desselben, in einer Packung umfasst, die Strahlung mit einer Wellenlänge von 300 bis 335 nm blockiert.

15. Pharmazeutische Zubereitung nach Anspruch 1, wässrige Zusammensetzung nach Anspruch 7, Verfahren nach Anspruch 13 oder Verfahren nach Anspruch 14, wobei Verbindung der Formel (1) Ripasudil ist.

## Revendications

1. Préparation pharmaceutique obtenue par stockage d'une composition aqueuse comprenant un composé représenté par la formule (1) : 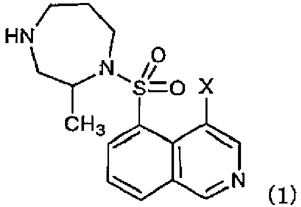
dans laquelle X représente un atome d'halogène,
ou un sel de celui-ci, ou un solvate du composé ou de son sel, dans un emballage qui bloque les rayons ayant une longueur d'onde de 300 à 335 nm.

2. Préparation pharmaceutique selon la revendication 1, dans laquelle l'intérieur de l'emballage est visible.

3. Préparation pharmaceutique selon la revendication 1 ou 2, dans laquelle un emballage primaire est un récipient comprenant une substance qui empêche la transmission de la lumière ultraviolette.

4. Composition pharmaceutique selon l'une quelconque des revendications 1 à 3, dans laquelle l'emballage primaire est un récipient comprenant un élément comprenant un substance qui empêche la transmission de la lumière ultraviolette.

5. Composition pharmaceutique selon l'une quelconque des revendications 1 à 4, dans laquelle l'emballage primaire est un récipient fait d'une résine à base de polyoléfine ou d'une résine à base de polyester.

6. Composition pharmaceutique selon l'une quelconque des revendications 1 à 5, qui comprend, en tant qu'emballage secondaire, un sac comprenant une substance qui empêche la transmission de la lumière ultraviolette.

7. Composition aqueuse comprenant un composé représenté par la formule (1) : 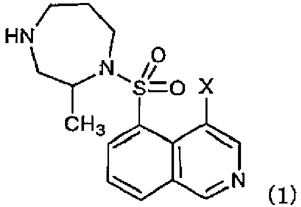
dans laquelle X représente un atome d'halogène,
ou un sel de celui-ci, ou un solvate du composé ou de son sel,
la composition aqueuse étant stockée dans un emballage qui bloque les rayons ayant une longueur d'onde de 300 à 335 nm.

8. Composition aqueuse selon la revendication 7, dans laquelle l'intérieur de l'emballage est visible.

9. Composition aqueuse selon la revendication 7 ou 8, dans laquelle un emballage primaire est un récipient comprenant une substance qui empêche la transmission de la lumière ultraviolette.

10. Composition aqueuse selon l'une quelconque des revendications 7 à 9, dans laquelle l'emballage primaire est un récipient comprenant un élément comprenant une substance qui empêche la transmission de la lumière ultraviolette.

11. Composition aqueuse selon l'une quelconque des revendications 7 à 10, dans laquelle l'emballage primaire est un récipient fait d'une résine à base de polyoléfine ou d'une résine à base de polyester.

12. Composition aqueuse selon l'une quelconque des revendications 7 à 11, qui comprend, en tant qu'emballage secondaire, un sac comprenant une substance qui empêche la transmission de la lumière ultraviolette.

13. Procédé pour améliorer la stabilité à la lumière d'un composé représenté par la formule (1) : 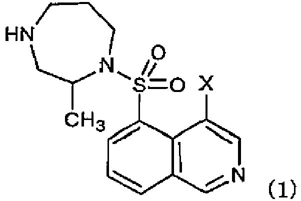
dans laquelle X représente un atome d'halogène,
ou d'un sel de celui-ci, ou d'un solvate du composé ou de son sel, dans une composition aqueuse,
le procédé comprenant l'étape de stockage de la composition aqueuse comprenant le composé représenté par la formule (1), ou un sel de celui-ci, ou un solvate du composé ou de son sel, dans un emballage qui bloque les rayons ayant une longueur d'onde de 300 à 335 nm.

14. Procédé pour conserver un composé représenté par la formule (1) : 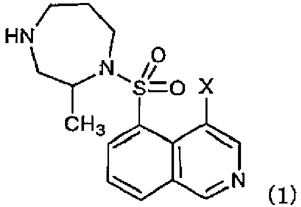
dans laquelle X représente un atome d'halogène, ou un sel de celui-ci, ou un solvate du composé ou de son sel,
le procédé comprenant l'étape de stockage d'une composition aqueuse comprenant le composé représenté par la formule (1), ou un sel de celui-ci, ou un solvate du composé ou de son sel, dans un emballage qui bloque les rayons ayant une longueur d'onde de 300 à 335 nm.

15. Préparation pharmaceutique selon la revendication 1, composition aqueuse selon la revendication 7, procédé selon la revendication 13, ou procédé selon la revendication 14, dans lequel le composé représenté par la formule (1) est le ripasudil.
